# EUROPÄISCHE PATENTSCHRIFT

(11) **EP 1 759 900 B1**
(45) Veröffentlichungstag und Bekanntmachung des Hinweises auf die Patenterteilung: **05.11.2008**
(21) Anmeldenummer: 06017729.2
(22) Anmeldetag: 25.08.2006
(51) Int. Cl.: B60H 3/00, A61L 9/12

(54) **Duftstoffgerät zur Anwendung in einem Kraftfahrzeug**
Fragrance dispenser for use in a vehicle
Appareil dispenseur de parfum pour utilisation dans un véhicule

(30) Priorität: 05.09.2005 DE 102005041989
(43) Veröffentlichungstag der Anmeldung: 07.03.2007
(73) Patentinhaber: GM Global Technology Operations, Inc., Detroit, MI 48265-3000 (US)
(72) Erfinder: Giordimaina, Karim, 65462 Ginsheim-Gustavsburg (DE); Lassota, Andreas Dr., 55437 Ockenheim (DE); Rudolph, Kai, 65199 Wiesbaden (DE)
(74) Vertreter: Strauss, Peter

(56) Entgegenhaltungen:
- DE-A1- 2 438 924
- DE-U1- 20 207 512
- FR-A- 2 373 291
- US-A- 3 006 042

## Beschreibung

Die Erfindung betrifft ein Duftstoffgerät zur Anwendung in einem Kraftfahrzeug, insbesondere einem Personenkraftfahrzeug, wobei das Gerät Öffnungen zum Ausgeben des Dufts aufweist, der von dem in dem Gerät angeordneten Duftstoff abgegeben wird.

Heutige Duftspender für Personenkraftfahrzeuge werden größtenteils über den Zubehörhandel vertrieben. Es gibt sie in Form von Behältnissen, die an die Lüftungsgitter eingeklipst werden. Weiterhin gibt es Duftspender, die als Granulat im Aschenbecher wirken oder als am Rückspiegel hängender "Duftbaum" Duftstoffe verbreiten.

Ein Duftstoffgerät der Eingangs genannten Art ist aus der DE 298 12 056 U1 bekannt. Bei diesem ist eine dosierte Abgabe des Duftstoffs möglich. Hierzu ist in einem Deckel ein Drehteller vorgesehen, der über Löcher im Deckel bewegt werden kann, sodass diese geöffnet oder geschlossen werden. Durch mehr oder minder weites Öffnen der jeweiligen Öffnung kann der Duftaustritt dosiert werden.

Es sind ferner dosierbare Duftstoffgeräte zur Anwendung in Personenkraftwagen bekannt. Ein dosierbares Duftstoffgerät ist in der DE 297 19 510 U1 beschrieben. Dort nimmt ein mittels zweier Klipse an einem Lüftungsgitter befestigbares Gehäuse einen Behälter für den Duftstoff auf. Die Austrittsöffnung des Behälters ist mittels eines Kegels verschließbar bzw. kann durch Veränderung der Position des Kegels mehr oder weniger freigegeben werden, sodass der Duftstoff dosiert aus dem Behälter austreten kann.

In der DE 202 07 512 U1 ist ein Duftspender für Kraftfahrzeuge beschrieben. Dieser weist einen zylinderförmigen Behälter auf, der an seinen beiden Stirnseiten erste Öffnungen hat und in seinem Inneren einen verdrehbaren Einsatz aufweist, der zweite Öffnungen hat, die mit den ersten Öffnungen des Behälters in eine korrespondierende Stellung bringbar sind.

Ein dosierbares Duftstoffgerät, das integraler Bestandteil der Luftdüse eines Fahrzeugs ist, ist in der DE 202 12 778 U1 beschrieben. In der DE 20 2004 010 015 U1 ist ein dosierbares Duftstoffgerät zur Anordnung in einem Personenkraftfahrzeug veranschaulicht, bei dem ein im Fahrzeuginneren angeordnetes Gehäuse eine um deren Lenksachse drehbare Kartusche aufnimmt, in die ein Duftstoffträger eingesetzt ist.

In der DE 90 05 101 U1 ist ein Duftstoffgerät zur Anwendung in einem Personenkraftfahrzeug beschrieben, dass ein einfaches Auswechseln des Duftstoffs nach dem Verbrauch ermöglicht. Das Duftstoffgerät besteht aus einer mit Perforationen versehenen Kapsel, in die der Duftstoff einbringbar ist. Die Kapsel ist zweiteilig, wobei die beiden Teile mittels eines Scharniers oder dergleichen verbunden sind. Der eine Teil der Kapsel weist eine ebene Rückwand auf, auf der, zur haftenden Anbringung der Kapsel, ein Klebestreifen angeordnet ist. Der andere Teil der Kapsel ist mit den Perforationen versehen und mittels einer Raste mit dem einen Teil verbindbar.

In der FR 2 373 291 A ist ein im Haushalt oder am Arbeitsplatz verwendbares Duftstoffgerät beschrieben, dessen oberer Bereich als Aschenbecher ausgebildet ist. Das Duftstoffgerät ist auch zur Anwendung in einem Kraftfahrzeug geeignet, so dass FR 2 373 291 als nächstliegender Stand der Technik gilt. Im unteren Bereich weist das Duftstoffgerät Öffnungen zum Ausgeben des Dufts auf, der von einem in den Duftstoffgerät angeordneten Duftstoff abgegeben wird. Das Duftstoffgerät besitzt ein rotationssymmetrisches Gehäuse mit einem Ringabschnitt sowie einen lösbar mit dem Gehäuse verbundenen Deckel. Im Gehäuse ist ein Aufnahmeraum für den Duftstoff gebildet. Das Gehäuse ist mit Öffnungen zur Ausgabe des Duftstoffs versehen.

Aufgabe der vorliegenden Erfindung ist es, ein Duftstoffgerät zur Anwendung in einem Kraftfahrzeug, insbesondere einem Personenkraftfahrzeug zu schaffen, dass sich durch einen einfachen Aufbau, ein ansprechendes Erscheinungsbild sowie durch eine vorteilhafte, ansprechende Anordnung im Fahrzeug auszeichnet. Es soll insbesondere möglich sein, ein Fahrzeug nachträglich mit dem Duftstoffgerät auszustatten, ohne das dies vom Nutzer des Fahrzeuges offensichtlich als Nachrüstung erkannt wird.

Gelöst wird die Aufgabe durch ein Duftstoffgerät der Eingangs genannten Art, dass gekennzeichnet ist durch ein rotationssymmetrisches Gehäuse, dass einen Ringabschnitt aufweist, sowie durch einen lösbar mit dem Gehäuse verbundenen Deckel, wobei im Gehäuse ein Aufnahmeraum für den Duftstoff gebildet ist, und das Gehäuse mit Öffnungen zum Ausgeben des Dufts versehen ist, wobei ferner das Duftstoffgerät in eine rotationssymmetrische Öffnung im Inneren des Fahrzeugs einsetzbar ist, wobei das Gehäuse einen Bodenabschnitt aufweist, sowie zwischen dem Bodenabschnitt und dem Deckel der Aufnahmeraum gebildet ist, wobei ferner das Gehäuse im Bereich des Ringabschnitts, auf der dem Deckel abgewandten Seite des Bodenabschnitts, und im Bereich des Bodenabschnitts mit den Öffnungen versehen ist.

Die erfindungsgemäße Gestaltung des Duftstoffgeräts, das die im Wesentlichen rotationssymmetrische Form aufweist, ermöglicht es, dieses in geeignete Öffnungen im Innenraum des Fahrzeugs einzubringen. Die jeweilige Öffnung zur Aufnahme des Duftstoffgeräts befindet sich insbesondere in der Instrumententafel des Fahrzeuges oder einer Konsole des Fahrzeuges. Es ist insbesondere an die Mittelkonsole des Fahrzeugs gedacht, in die das Duftstoffgerät seitlich oder mittig eingesteckt werden kann. Als besonders vorteilhaft wird die Anordnung des Duftstoffgeräts an solchen Orten des Fahrzeuginnenraums angesehen, die ohnehin der Aufnahme rotationssymmetrischer Körper dienen, die eine definierte Funktion im Fahrzeug besitzen. So sind diverse Fahrzeuge mit einem so genannten "Cup-Holder" ausgerüstet, der demzufolge die Aufgabe hat, eine Flasche, eine Getränkedose, eine Tasse oder dergleichen aufzunehmen. Solche "Cup-Holder" sind genauso geeignet, das erfindungsgemäße, rotationssymmetrisch gestaltete Duftstoffgerät aufzunehmen. Auch der Raum, der in einem Fahrzeug beispielsweise der Aufnahme eines rund gestalteten Aschenbechers dient, ist, sofern das Fahrzeug nicht mit einem Aschenbecher ausgestattet werden soll, geeignet, das Duftstoffgerät aufzunehmen.

Das Gehäuse des Duftstoffgerätes ist so gestaltet, dass es im Bereich des Ringabschnitts und eines Bodenabschnitts des Gehäuses die Öffnungen aufweist. Der vom Duft stoff abgegebene Duft gelangt somit durch die Öffnungen des Bodenabschnitts und gegebenenfalls durch die Öffnungen des Ringabschnitts nach außen.

Es wird als besonders vorteilhaft angesehen, wenn die Duftabgabe des Duftstoffgerätes dosierbar ist. Dies kann bei dem erfindungsgemäßen Duftstoffgerät auf besonders einfache Art und Weise dadurch verwirklicht werden, dass in das Gehäuse ein rotationssymmetrisches Einsatzteil einsetzbar ist, wobei das Einsatzteil mit Öffnungen zum Ausgeben des Duftes versehen ist. Der Öffnungsquerschnitt zur Ausgabe des Duftstoffs wird vorzugsweise durch rotatorische oder translatorische Relativbewegungen von Gehäuse und Einsatzteil eingestellt.

Unter dem Aspekt einer rotatorischen Relativbewegung von Gehäuse und Einsatzteil wird es als besonders vorteilhaft angesehen, wenn das Einsatzteil bezüglich dessen Rotationsachse relativ zum Gehäuse drehbar ist, zum Anordnen von Gehäuse und Einsatzteil in einer die Ausgabe des Duftstoffs unterbrechenden Schließstellung und einer die Ausgabe des Duftstoffs gestattenden Öffnungsstellung der Öffnungen in Gehäuse und Einsatzteil. Durch Herbeiführen von Zwischenstellungen kann das Einsatzteil die Öffnungen im Gehäuse mehr oder weniger abdecken.

Besonders einfach lässt sich die Dosierungsfunktion des Einsatzteils verwirklichen, wenn das Einsatzteil im Bereich dessen Ringabschnitt und dessen Bodenabschnitt mit den Öffnungen versehen ist.

Neben einer eventuell bevorzugten Gestaltung des Duftstoffgerätes mit der Topfform, womit dem Duftstoffgerät die Zusatzfunktion des Cup-Holders zukommt, ist es durchaus denkbar, ausschließlich die Duftabgabefunktion durch das Duftstoffgerät zu verwirklichen. In diesem Fall kann die Gestaltung des Duftstoffgeräts wesentlich freier gewählt werden. Als vorteilhafte Gestaltung, die ästhetisch besonders ansprechend ist, wird die Form eines Zylinders mit auf diesem aufgesetzten Kegelstumpf angesehen. In im Bereich der Rotationsachse des Kegelstumpfes geneigt angeordneten Wandlungen des Kegelstumpfs sind die Öffnungen zum Durchtritt des Duftstoffs angeordnet. In Art des vorbeschriebenen Einsatzteils kann mit dem kegelstumpfförmigen Gehäuse ein zweiter Körper zusammenwirken, der, auf Grund der Relativbewegung von Gehäuse und Einsatzteil, die dosierte Abgabe des Duftstoffs ermöglicht.

Der Aufnahmeraum, der zwischen dem Bodenabschnitt des Gehäuses und dem insbesondere unterhalb des Bodenabschnitts angeordneten Deckel gebildet ist, dient beispielsweise der Aufnahme eines Trägerstoffs mit in diesem eingelagerten Duftstoff. Bei dem Trägerstoff mit in diesem eingelagerten Duftstoff handelt es sich beispielsweise um ein Duftgel oder eine Duftseife. Es sind aber selbstverständlich auch andere Aufbewahrungsarten für den Duftstoff möglich, dieser kann durchaus auch eine Flüssigkeit sein.

Die einzelnen Teile des Duftstoffgerätes, somit insbesondere das Gehäuse und das Einsatzteil, bestehen insbesondere aus Kunststoff und sind vorzugsweise durch Spritzgießen hergestellt. Diese Teile sind auf einfache Art und Weise einer besonders vorteilhaften ästhetischen Gestaltung zugänglich, sei es durch die Art der Oberflächengestaltung oder die Farbe. Die harmonische und integrative Gestaltung ermöglicht es den "After Sales-Charakter" zu eliminieren. Es ist durchaus denkbar, diese Teile des Duftstoffgerätes so zu gestalten, dass sie in Glas- oder Edelsteinart und überdies in Farben schimmern können, sodass sich für das Duftstoffgerät ein "Juwel-Effekt" ergibt.

Bevorzugte Weiterbildungen der Erfindung ergeben sich aus den Unteransprüchen und der nachfolgenden Beschreibung.

Nachfolgend werden mehrere Ausführungsbeispiele der Erfindung, ohne hierauf beschränkt zu sein, unter Bezugnahme auf die Zeichnung näher erläutert. In der Zeichnung zeigt:
- Figur 1: eine räumliche Ansicht eines Teilbereiches einer Mittelkonsole eines Personenkraftwagens mit einem vor einem Schalthebel angeordneten Cup-Holder,
- Figur 2: eine erste Ausführungsform des erfindungsgemäßen Duftstoffgeräts in einer räumlichen Ansicht,
- Figur 3: ein Schnitt durch das Duftstoffgerät gemäß Figur 2, in Verbindung mit dem in Figur 1 gezeigten Cup-Holder,
- Figur 4: eine zweite Ausführungsform des Duftstoffgeräts in einer Schnittdarstellung gemäß Figur 3, bei einer Dosierstellung, in der eine maximale Duftabgabe stattfindet,
- Figur 5: einen Schnitt durch das Duftstoffgerät gemäß der Linie V-V in Figur 4,
- Figur 6: einen Schnitt gemäß Figur 5 für einen Zustand des Duftstoffgerätes, bei dem keine Duftstoffabgabe erfolgt und
- Figur 7: eine dritte Ausführungsform des Duftstoffgeräts, bei kegelstumpfförmiger Gestaltung.

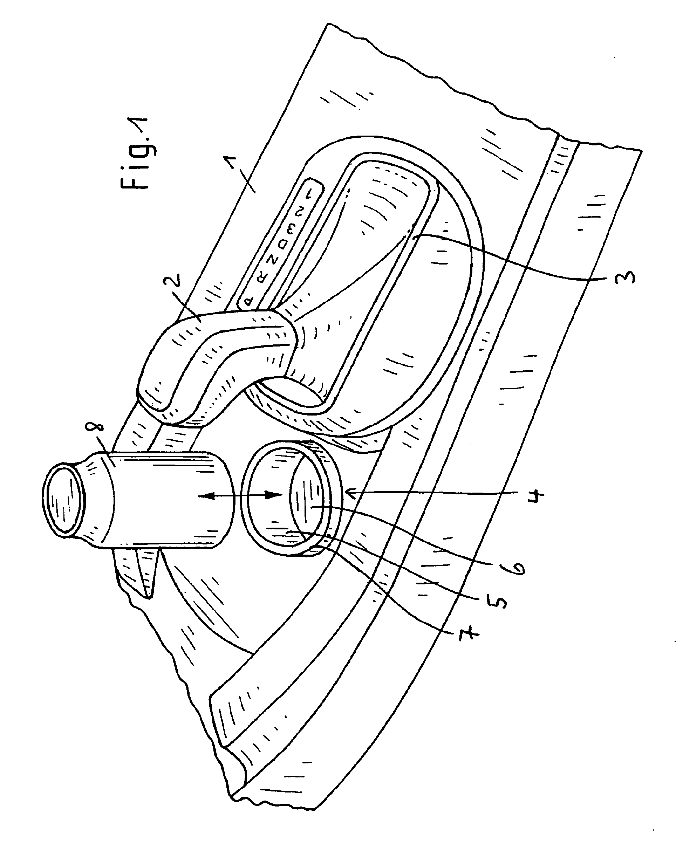

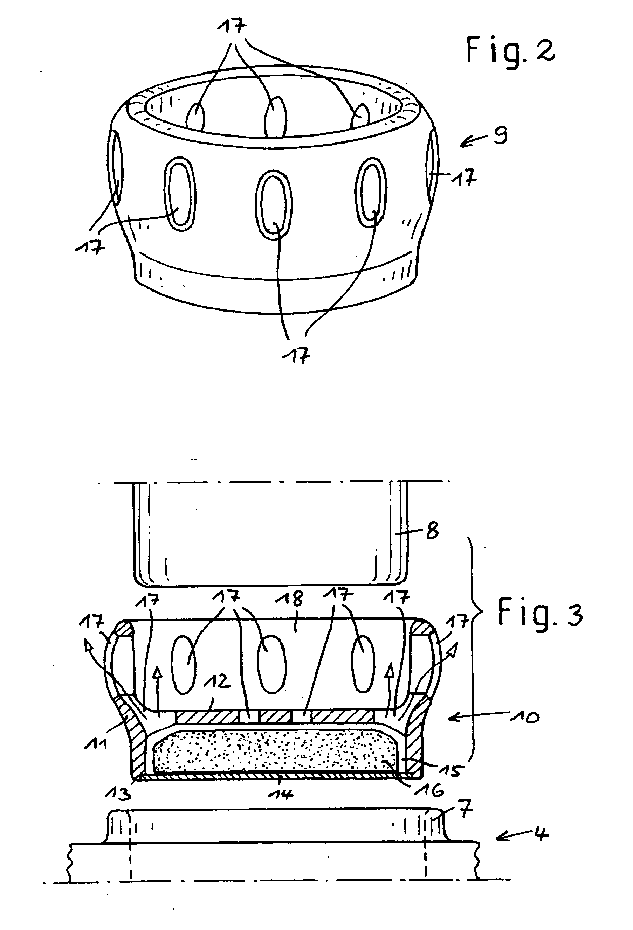

Figur 1 zeigt einen Teilausschnitt einer Mittelkonsole 1 eines Personenkraftfahrzeuges, gesehen über eine Teillänge der Mittelkonsole 1. Der dargestellte Bereich veranschaulicht den Schalthebel 2 des automatischen Schaltgetriebes mit dem dieses umgebenden Wulst 3. In Fahrtrichtung gesehen vor dem Schalthebel 2 bzw. Wulst 3 ist die Mittelkonsole 1 mit einer Öffnung versehen, in die ein rotationssymmetrischer Topf 4 eingesetzt ist, der die Funktion eines Cup-Holders hat. Der Topf 4 weist einen sich über einen Vollkreis erstreckenden Ringabschnitt 5, einen unteren Bodenabschnitt 6 und einen oberen Wulstabschnitt 7 auf, dessen Außendurchmesser größer ist als der Durchmesser der Öffnung. In den Topf ist eine Getränkedose 8 im Sinne des veranschaulichten Doppelpfeils einsteckbar bzw. aus dem Topf herausnehmbar. Der Außendurchmesser der Dose 8 ist geringfügig geringer als der Innendurchmesser des Ringabschnitts 5. Statt der Dose 8 ist in den Topf 4 das in Figur 2 gezeigte Duftstoffgerät 9 einsteckbar, dass, wie es der Figur 3 zu entnehmen ist, gleichfalls der Aufnahme der Dose 8 dient, womit das Duftstoffgerät 9 die Zusatzfunktion des Cup-Holders besitzt.

Wie der Darstellung der Figuren 2 und 3 zu entnehmen ist, weist das Duftstoffgerät 9 ein rotationssymmetrisches Gehäuse 10 auf, dass einen Ringabschnitt 11 und einen Bodenabschnitt 12 aufweist. Der Bodenabschnitt 12 ist in deutlichem Abstand zur unteren Umlaufkante 13 des Gehäuses 10 angeordnet und es ist das Gehäuse 10 im Bereich der Umlaufkante 13 mit einer Aufnahme für einen lösbar mit dem Gehäuse 10 verbindbaren Deckel 14 versehen. Dieser Deckel 14 lässt sich beispielsweise in das Gehäuse 10 einklemmen oder aber mittels nicht veranschaulichter Rastmittel rastieren. Es ist auch denkbar, eine von außen lösbare Verbindung von Boden 14 und Gehäuse 10 durch eine Bajonettverschluss-Anordnung herzustellen.

Zwischen dem Bodenabschnitt 12 und dem Deckel 14 ist ein Aufnahmeraum 15 für den Duftstoff 16 gebildet. Dieser ist als Trägerstoff gestaltet, in den der Duftstoff eingelagert ist. Der Trägerstoff mit Duftstoff weist gleichfalls eine rotationssymmetrische Gestaltung auf.

Das Gehäuse 10 ist sowohl im Bereich seines Bodenabschnitts 12 als auch in dem sich oberhalb des Bodenabschnitts 12 erstreckenden Bereich des Ringabschnitts 11 mit Öffnungen 17 versehen. Sowohl durch die Öffnungen 17 im Bodenabschnitt 12 als auch durch die Öffnungen 17 im Ringabschnitt 11 kann Duft aus dem Aufnahmeraum 15 gelangen. Ist die Dose 8 in den oberhalb des Bodenabschnitts 12 gebildeten oberen Aufnahmeraum 18 des Gehäuses 10 eingesetzt, deckt die Dose 8 die Öffnungen 17 im Bodenabschnitt 12 ab, sodass der Duft nur durch die im Ringabschnitt 11 angeordneten Öffnungen 17 austreten kann.

Die Explosionsdarstellung nach Figur 3 zeigt das geschnitten dargestellte Duftstoffgerät 9 vor dem Einsetzen in den in der Mittelkonsole 1 gelagerten Topf 4, ferner die Dose 8 vor dem Einsetzen in das Duftstoffgerät 9.

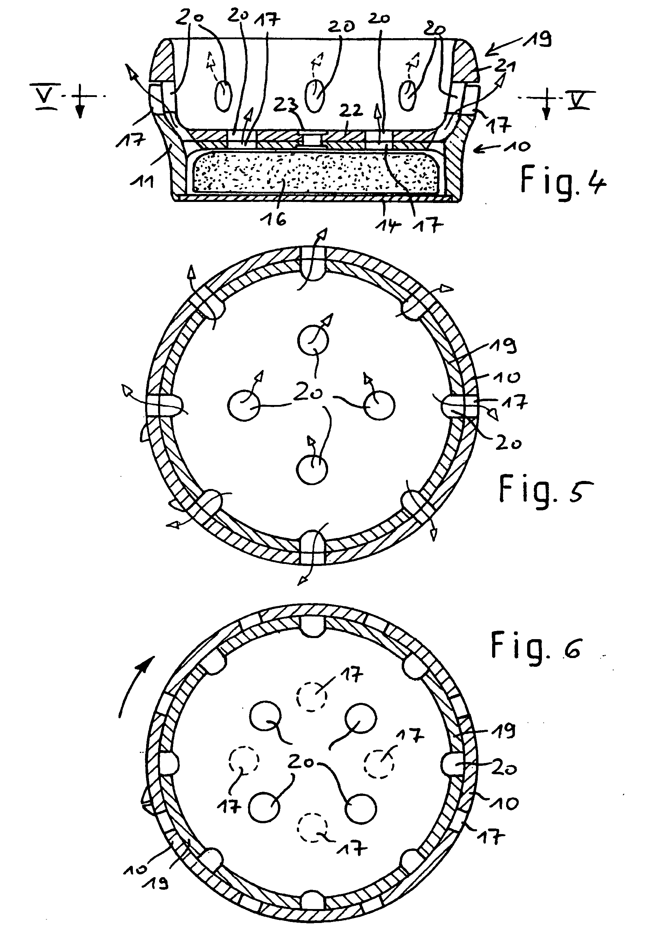

Die Ausführungsform nach den Figuren 4 bis 6 ist gegenüber der Ausführungsform nach den Figuren 2 und 3 insofern modifiziert, dass in das Gehäuse 10 ein rotationssymmetrisches Einsatzteil 19 eingesteckt ist. Das Einsatzteil 19 ist bezüglich dessen Rotationsachse relativ zum Gehäuse 10 drehbar. Es weist gleichfalls Öffnungen, die dort mit der Bezugsziffer 20 versehen sind, zum Ausgeben des Duftstoffs 16 auf. Diese Öffnungen 20 sind entsprechend in den Ringabschnitt 21 und den Bodenabschnitt 22 des Einsatzteils 19 eingebracht. Figur 5 veranschaulicht eine Drehstellung von Gehäuse 10 und Einsatzteil 19, in der die Öffnungen 17 und 20 dieser beiden Teile miteinander fluchten, sodass der Duftstoff durch diese Öffnungen austreten kann. Figur 6 veranschaulicht eine Drehstellung von Gehäuse 10 und Einsatzteil 19, in der die Öffnungen 17 und 20 der beiden Teile nicht miteinander fluchten, somit der Ringabschnitt 21 und der Bodenabschnitt 22 mit denjenigen Wandungsbereiche, die nicht die Öffnungen 20 aufweisen, die Öffnungen 17 im Gehäuse 10 abdecken. Durch mehr oder weniger starkes Abblenden der Öffnungen 17 auf Grund der entsprechenden Drehstellung des Einsatzteils 19 relativ zum Gehäuse 10, ist eine dosierte Abgabe des Duftstoffs aus dem Duftstoffgerät 9 möglich.

Figur 4 veranschaulicht, dass das Gehäuse 10 und das Einsatzteil 19 durchaus mittels eines Zapfens 23 miteinander unlösbar verbunden sein können. Es reicht aber völlig aus, eine lösbare Anordnung vorzusehen, somit das einfache Einstecken des Einsatzteils 19 in das Gehäuse 10. Bei der veranschaulichten Ausführungsform mit dem Einsatzteil 19 sind die Löcher 17 im Ringabschnitt 11 des Gehäuses 10 nicht geschlossen, sondern oben offen; die obere Begrenzung dieser Öffnungen 17 erfolgt durch den oberen Bereich des Ringabschnitts 21 des Einsatzteils 19.

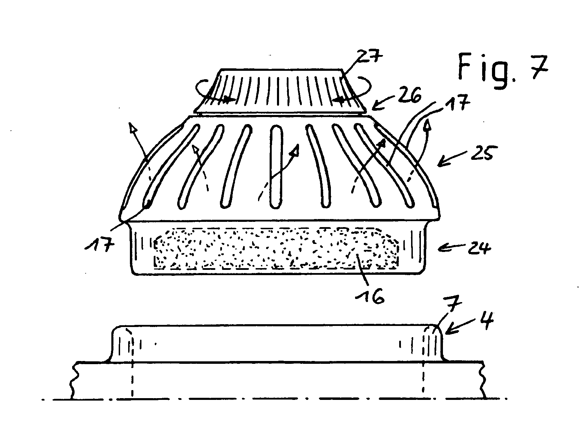

In der Figur 7 ist eine Ausführungsform des Duftstoffgerätes 9 veranschaulicht, der nicht die Zusatzfunktion des Cup-Holders zukommt. Das Duftstoffgerät 9 ist, wie vorbeschrieben, in den Topf 4 einsetzbar. An den zylindrischen Abschnitt 24 des Duftstoffgerätes 9, wie er zu den beiden ersten Ausführungsformen der Erfindung wiedergegeben ist und zwischen dem Bodenabschnitt 12 und dem Deckel 14 gebildet ist, schließt sich ein kegelstumpfförmiger Abschnitt 25 an. Dieser kegelstumpfförmiger Abschnitt 25 entspricht dem Ringabschnitt 11, der bei der anderen Ausführungsform mit den Öffnungen 17 versehen ist. Die bei dieser Ausführungsform gleichfalls im Bodenabschnitt 12 vorhandenen Öffnungen 17 sind nicht mitveranschaulicht. In das Gehäuse 10 ist ein Einsatzteil 26 eingesetzt, dessen oben aus dem Gehäuse 10 ragender Abschnitt 27 die Funktion eines Griffs zum Drehen des Einsatzteils 26 aufweist. Das Einsatzteil 26 erstreckt sich bis zum Bodenabschnitt 12 des Gehäuses 10 und ist mit einem in der Figur nicht veranschaulichten Ringabschnitt versehen, der Öffnungen aufweist, die entweder in fluchtende Stellung mit den Öffnungen 17 des Gehäuses, zwecks Duftaustritt, oder in eine diese Öffnungen 17 abdeckende Stellung drehbar ist.

### Bezugszeichenliste

- Mittelkonsole: 1
- Schalthebel: 2
- Wulst: 3
- Topf: 4
- Ringabschnitt: 5
- Bodenabschnitt: 6
- Wulstabschnitt: 7
- Dose: 8
- Duftstoffgerät: 9
- Gehäuse: 10
- Ringabschnitt: 11
- Bodenabschnitt: 12
- Umlaufkante: 13
- Deckel: 14
- Aufnahmeraum: 15
- Duftstoff: 16
- Öffnung: 17
- Aufnahmeraum: 18
- Einsatzteil: 19
- Öffnung: 20
- Ringabschnitt: 21
- Bodenabschnitt: 22
- Zapfen: 23
- zylindrischer Abschnitt: 24
- kegelstumpfförmiger Abschnitt: 25
- Einsatzteil: 26
- Abschnitt: 27

## Patentansprüche

1. Duftstoffgerät (9) zur Anwendung in einem Kraftfahrzeug, insbesondere einem Personenkraftfahrzeug, wobei das Duftstoffgerät (9) Öffnungen (17) zum Ausgeben des Dufts , der von dem in dem Duftstoffgerät (9) angeordneten Duftstoff (16) abgegeben wird, ein rotationssymmetrisches Gehäuse (10), das einen Ringabschnitt (11) aufweist, sowie einen lösbar mit dem Gehäuse (10) verbundenen Deckel (14) aufweist, wobei im Gehäuse (10) ein Aufnahmeraum (15) für den Duftstoff (16) gebildet ist, und das Gehäuse (10) mit den Öffnungen (17) zur Ausgabe des Duftstoffes (16) versehen ist, wobei ferner das Duftstoffgerät (9) in eine rotationssymmetrische Öffnung (4) im Innenraum des Fahrzeugs einsetzbar ist, **dadurch gekennzeichnet, dass** das Gehäuse (10) einen Bodenabschnitt (12) aufweist, wobei zwischen dem Bodenabschnitt (12) und dem Deckel (14) der Aufnahmeraum (15) gebildet ist, wobei ferner das Gehäuse (10) im Bereich des Ringabschnitts (11), auf der dem Deckel (14) abgewandten Seite des Bodenabschnitts (12), und im Bereich des Bodenabschnitts (12) mit den Öffnungen (17) versehen ist.

2. Duftstoffgerät nach Anspruch 1, **dadurch gekennzeichnet, dass** in das Gehäuse (10) ein rotationssymmetrisches Einsatzteil (19, 26) einsetzbar ist, wobei das Einsatzteil (19, 26) mit Öffnungen (20) zum Ausgeben des Duftstoffs (16) versehen ist.

3. Duftstoffgerät nach Anspruch 2, **dadurch gekennzeichnet, dass** das Einsatzteil (19, 26) bezüglich dessen Rotationsachse relativ zum Gehäuse (10) drehbar ist, zum Anordnen von Gehäuse (10) und Einsatzteil (19, 26) in einer die Ausgabe des Duftstoffs (16) unterbrechenden Schließstellung und einer die Ausgabe des Duftstoffs (16) gestattenden Öffnungsstellung der Öffnungen (17, 20) im Gehäuse (10) und im Einsatzteil (19, 26).

4. Duftstoffgerät nach Anspruch 2 oder 3, **dadurch gekennzeichnet, dass** das Einsatzteil (19, 26) im Bereich dessen Ringabschnitt (21) und dessen Bodenabschnitt (22) mit den Öffnungen (20) versehen ist.

5. Duftstoffgerät nach einem der Ansprüche 1 bis 4, **dadurch gekennzeichnet, dass** es in eine Öffnung in einer Konsole oder der Instrumententafel des Fahrzeugs, insbesondere eine Öffnung in der Mittelkonsole (1) des Fahrzeugs, oder in eine Öffnung eines in die Konsole oder Instrumententafel des Fahrzeugs integrierten Cup-Holders (4) einsetzbar ist.

6. Duftstoffgerät nach einem der Ansprüche 1 bis 5, **dadurch gekennzeichnet, dass** es in Art eines Cup-Holders gestaltet ist, derart, dass das Gehäuse (10) und/oder das Einsatzteil (19) eine Topfform bilden.

7. Duftstoffgerät nach einem der Ansprüche 1 bis 6, **dadurch gekennzeichnet, dass** es die Form eines Zylinders (24), mit auf diesen aufgesetztem Kegelstumpf (25) aufweist.

8. Duftstoffgerät nach einem der Ansprüche 1 bis 7, **dadurch gekennzeichnet, dass** der Aufnahmeraum (15) der Aufnahme eines Trägerstoffs mit in diesem eingebetteten Duftstoff (16) dient.

## Claims

1. Fragrance dispenser (9) to be applied in a motor vehicle, primarily in a passenger car, wherein the fragrance dispenser (9) is equipped with openings (17) to dispense the fragrance which will be dispensed by the fragrance (16) contained in the fragrance dispenser (16), a rotation symmetric box (10) equipped with a ring section (11) and with a cover (14) flexibly connected with the box (10), wherein in the box (10) there is an acceptance area (15) for the fragrance (16), and the box (10) is equipped with the openings (17) to dispense the fragrance (16), wherein in addition the fragrance dispenser (9) can be placed into a rotation symmetric opening (4) in the internal area of the motor vehicle, **characterized**
**in that** the box (10) has a basement area (12), wherein between the basement area (12) and the cover (14) there is the acceptance area (15), wherein in addition the box (10) in the area of the ring section (11) and on the side of the basement area (12) turned away from the cover (14) is equipped with openings (17).

2. Fragrance dispenser according to claim 1, **characterized in that** in the box (10) you can place a rotation symmetric insertion component (19, 26), wherein the insertion component (19, 26) is equipped with openings (20) to dispense the fragrance (16).

3. Fragrance dispenser according to claim 2, **characterized in that** the insertion component (19, 26) can be rotated to its axis of rotation relating to the box (10), in order to align the box (10) and the insertion component (19, 26) in a closing position interrupting the dispensation of the fragrance (16) and an open position of the openings (17, 20) in the box (10) and in the insertion component (^19, 26) permitting the dispensation of the fragrance (16).

4. Fragrance dispenser according to claim 2 or 3, **characterized in that** the insertion component (19, 26) is equipped with the openings (20) in its ring area (21) and in its basement area (22).

5. Fragrance dispenser according to one of the claims 1 to 4, **characterized in that** it can be placed into one opening in a console or instrument panel of the motor vehicle, primarily into an opening in the intermediate console (1) of the motor vehicle, or into an opening of cup holder (4) integrated in the console or instrument panel of the motor vehicle.

6. Fragrance dispenser according to one of the claims 1 to 5, **characterized in that** it is designed in the shape of a cup holder, so that the box (10) and/or the insertion component (19) form the shape of a pot.

7. Fragrance dispenser according to one of the claims 1 to 6, **characterized in that** it has the shape of a cylinder (24) with a truncated cone (25) put on it.

8. Fragrance dispenser according to one of the claims 1 to 7, **characterized in that** the acceptance area (15) serves as acceptance of a carrier with the fragrance (16) embedded in it.

## Revendications

1. Diffuseur d'arômes (9) à utiliser dans un véhicule, en particulier dans un véhicule de tourisme, où le diffuseur d'arômes (9) est pourvu d' ouvertures (17) afin de répandre le parfum qui est contenu dans le diffuseur (9) de l'arôme (16), un boîtier à rotation symétrique (10), qui présente une fente en forme d'anneau (11) ainsi qu'un couvercle (14) relié au boîtier séparé (10); dans ce boîtier (10), on y trouve un espace d'absorption (15) pour la substance odorante (16), et le boîtier (10) est muni d'ouvertures (17) pour l'émission de la matière odorante (16); on peut mettre par ailleurs l'appareil de matière odorante (9) dans une ouverture à rotation symétrique (4) à l'intérieur du véhicule, **caractérisé en ce que** le boîtier (10) présente une section de fond (12); on trouve l'espace d'absorption (15) entre la section de fond (12) et le couvercle (14); par ailleurs le boîtier (10) est muni d'un couvercle (14) dans la zone de section en forme d'anneau (11), sur la partie adverse de la section de fond (12), et dans la zone de section de fond (12), d'ouvertures (17).

2. Diffuseur d'arômes selon revendication 1, **caractérisé en ce que** l'on puisse mettre dans le boîtier (10) une garniture de joint à rotation symétrique (19, 26), où cette garniture de joint est pourvue d'ouvertures (20) pour l'émission de la matière odorante (16).

3. Diffuseur d'arômes selon revendication 1, **caractérisé en ce que** la garniture de joint (19, 2 6) puisse tourner sur son axe de rotation par rapport au boîtier (10), pour le rangement du boîtier (10) et de la garniture de joint (19, 26) dans une position de fermeture interrompant l'émission de la substance odorante (16), et dans une position d'ouverture permettant l'émission de la substance odorante (16) des ouvertures (17, 20) dans le boîtier (10) et dans la garniture de joint (19, 26).

4. Diffuseur d'arômes selon revendications 2 et 3, **caractérisé en ce que** la garniture de joint (19, 26) est munie d'ouvertures (20) dans la zone de section en forme d'anneau (21) et la section de fond (22).

5. Diffuseur d'arômes selon revendications 1 à 4, **caractérisé en ce que** l'on puisse mettre un Cup-Holder intégré (4) dans une ouverture de console ou du tableau de bord du véhicule, en particulier une ouverture dans la console du milieu (1) du véhicule, ou dans une ouverture de la console ou du tableau de bord du véhicule.

6. Diffuseur d'arômes selon revendications 1 à 5, **caractérisé en ce qu'**il est présenté à la manière d'un Cup-Holder, de façon à ce que le boîtier (10) et / ou la garniture de joint (19) soient constitués en forme de pot.

7. Diffuseur d'arômes selon revendications 1 à 6, **caractérisé en ce qu'**il se présente sous forme de cylindre (24), avec par dessus un tronc conique (25).

8. Diffuseur d'arômes selon revendications 1 à 7, **caractérisé en ce que** l'espace d'absorption (15) sert à l'absorption d'un excipient dans la substance odorante incorporée (16).
